(19) Europäisches Patentamt
European Patent Office
Office européen des brevets

(11) Veröffentlichungsnummer: **0 569 794 A1**

(12) # EUROPÄISCHE PATENTANMELDUNG

(21) Anmeldenummer: **93106987.6**

(51) Int. Cl.⁵: **C07D 401/10**, A61K 31/47

(22) Anmeldetag: **29.04.93**

(30) Priorität: **12.05.92 FR 4215588**

(43) Veröffentlichungstag der Anmeldung:
**18.11.93 Patentblatt 93/46**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Dressel, Jürgen, Dr.**
**Claudiusweg 9**
**W-5600 Wuppertal 1(DE)**
Erfinder: **Fey, Peter, Dr.**
**Am Eickhof 23**
**W-5600 Wuppertal 1(DE)**
Erfinder: **Hanko, Rudolf H., Dr.**
**Schillerstrasse 23**
**W-4000 Düsseldorf 1(DE)**
Erfinder: **Hübsch, Walter, Dr.**
**Wildsteig 22**
**W-5600 Wuppertal 1(DE)**
Erfinder: **Krämer, Thomas, Dr.**
**In den Birken 92a**
**W-5600 Wuppertal 1(DE)**
Erfinder: **Müller, Ulrich E., Dr.**
**Claudiusweg 5**
**W-5600 Wuppertal 1(DE)**
Erfinder: **Müller-Gliemann, Matthias, Dr.**
**Laibacher Strasse 10**
**W-5650 Solingen-Ohligs(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal 1(DE)**
Erfinder: **Wohlfeil, Stefan, Dr.**
**Tucherweg 25**
**W-4010 Hilden(DE)**
Erfinder: **Knorr, Andreas, Dr.**
**Trillser Graben 10**
**W-4006 Erkrath 2(DE)**
Erfinder: **Stasch, Johannes-Peter, Dr.**
**Alfred-Nobel-Strasse 109**
**W-42651 Solingen(DE)**

(54) **Biphenylmethyl-substituierte Pyridone.**

(57) Biphenylmethyl-substituierte Pyridone werden durch Umsetzung von Pyridonen mit entsprechenden Biphenylmethyl-Verbindungen hergestellt.

Die Biphenylmethyl-substituierten Pyridone können als Wirkstoffe in Arzneimittel eingesetzt werden, insbesondere zur Behandlung von arterieller Hypertonie und Atherosklerose.

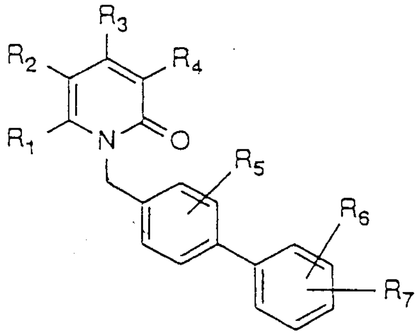

(I),

EP 0 569 794 A1

Die vorliegende Erfindung betrifft Biphenylmethyl-substituierte Pyridone, ein Verfahren zu ihrer Herstellung sowie ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksenkenden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die vorliegende Erfindung betrifft Biphenylmethyl-substituierte Pyridone der allgemeinen Formel (I)

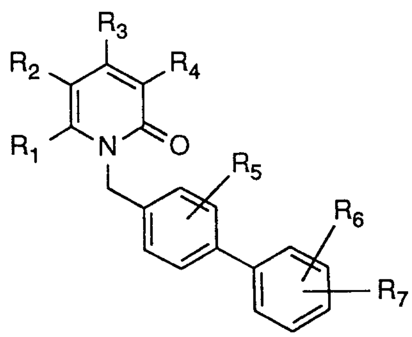

(I),

in welcher

| | |
|---|---|
| $R^1$ und $R^2$ | gleich oder verschieden sind und für Wasserstoff, Cyano, Halogen oder für geradkettiges oder verzweigtes Alkyl, Alkenyl oder Alkinyl mit jeweils bis zu 8 Kohlenstoffatomen stehen, die gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen oder Phenyl substituiert sind, oder für Cycloalkyl mit 3 bis 6 Kohlenstoffatomen stehen, für geradkettiges oder verzweigtes Acyl oder Alkoxycarbonyl mit jeweils bis zu 8 Kohlenstoffatomen, Benzyloxycarbonyl oder Carboxy stehen, oder für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder für die Gruppe der Formel $-CO-NR^8R^9$, $B-R^{10}$ oder $-NR^{11}R^{12}$ stehen, worin |
| $R^8$ und $R^9$ | gleich oder verschieden sind und Wasserstoff, Phenyl, geradkettiges oder verzweigtes Alkyl mit bis zu 6 Kohlenstoffatomen oder Benzyl bedeuten, |
| B | ein Sauerstoff- oder Schwefelatom bedeutet, |
| $R^{10}$ | geradkettiges oder verzweigtes Alkyl mit bis zu 8 Kohlenstoffatomen bedeutet, |
| $R^{11}$ und $R^{12}$ | gleich oder verschieden sind und die oben angegebene Bedeutung von $R^8$ und $R^9$ haben oder $R^{11}$ oder $R^{12}$ die $-SO_2R^{13}$-Gruppe bedeutet, worin |
| $R^{13}$ | geradkettiges oder verzweigtes Alkyl mit bis zu 6 Kohlenstoffatomen, Benzyl oder Phenyl bedeutet, die gegebenenfalls durch Methyl substituiert sind, |
| $R^3$ und $R^4$ | unter Einbezug der Doppelbindung einen Phenyl- oder Pyridylring ausbilden, der gegebenenfalls bis zu 3-fach gleich oder verschieden durch Hydroxy, Formyl, Carboxy, Halogen, geradkettiges oder verzweigtes Acyl oder Alkoxycarbonyl mit jeweils bis zu 8 Kohlenstoffatomen, geradkettiges oder verzweigtes Perfluoralkyl mit bis zu 6 Kohlen- |

stoffatomen oder durch geradkettiges oder verzweigtes Alkyl mit bis zu 8 Kohlenstoffatomen substituiert ist, das seinerseits durch Hydroxy oder geradkettiges oder verzweigtes Alkoxy mit bis zu 6 Kohlenstoffatomen substituiert sein kann, oder durch die Gruppe der Formel $-CONR^8R^9$ substituiert sind,

worin

$R^8$ und $R^9$ die oben angegebene Bedeutung haben,

$R^5$ und $R^6$ gleich oder verschieden sind und für Wasserstoff, Halogen oder geradkettiges oder verzweigtes Alkyl mit bis zu 8 Kohlenstoffatomen, oder für geradkettiges oder verzweigtes Perfluoralkyl mit bis zu 6 Kohlenstoffatomen stehen,

$R^7$ für einen Rest der Formel

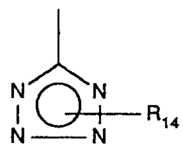

oder $CO-R^{15}$ steht,

worin

$R^{14}$ Wasserstoff, geradkettiges oder verzweigtes Alkyl mit bis zu 6 Kohlenstoffatomen oder Triphenylmethyl bedeutet

und

$R^{15}$ Hydroxy, geradkettiges oder verzweigtes Alkoxy mit bis zu 8 Kohlenstoffatomen, Phenoxy oder eine Gruppe der Formel $-NR^{16}R^{17}$ bedeutet,

worin

$R^{16}$ und $R^{17}$ gleich oder verschieden sind und Wasserstoff oder geradkettiges oder verzweigtes Alkyl mit bis zu 6 Kohlenstoffatomen oder Phenyl bedeuten,

und deren Salze.

Die erfindungsgemäßen Biphenylmethyl-substituierten Pyridone können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Biphenylmethyl-substituierten Pyridone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium-oder Calciumsalze, sowie Ammoniumsalze, die abgeleitet sind von Ammoniak oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Bevorzugt sind Verbindungen der allgemeinen Formel (I),

in welcher

$R^1$ und $R^2$ gleich oder verschieden sind und für Wasserstoff, Cyano, Chlor oder

für geradkettiges oder verzweigtes Alkyl, Alkenyl oder Alkinyl mit jeweils bis zu 6 Kohlenstoffatomen stehen, die gegebenenfalls durch Cyclopropyl, Cyclopentyl, Cyclohexyl, Hydroxy oder durch geradkettiges oder verzweigtes Alkoxy mit bis zu 4 Kohlenstoffatomen oder Phenyl substituiert sind, oder

für Cyclopropyl, Cyclopentyl oder Cyclohexyl stehen,

für geradkettiges oder verzweigtes Acyl oder Alkoxycarbonyl mit jeweils bis zu 6 Kohlenstoffatomen, Benzyloxycarbonyl oder Carboxy stehen, oder

für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder

für eine Gruppe der Formel $-CO-NR^8R^9$, $B-R^{10}$ oder $-NR^{11}R^{12}$ stehen,

worin

$R^8$ und $R^9$ gleich oder verschieden sind und Wasserstoff, Phenyl, geradkettiges oder verzweigtes Alkyl mit bis zu 4 Kohlenstoffatomen oder Benzyl bedeuten,

B ein Sauerstoff- oder Schwefelatom bedeutet,

$R^{10}$ geradkettiges oder verzweigtes Alkyl mit bis zu 6 Kohlenstoffatomen bedeutet,

$R^{11}$ und $R^{12}$ gleich oder verschieden sind und die oben angegebene Bedeutung von $R^8$ und $R^9$ haben oder $R^{11}$ oder $R^{12}$ die $-SO_2R^{13}$-Gruppe bedeutet,

worin

$R^{13}$ geradkettiges oder verzweigtes Alkyl mit bis zu 4 Kohlenstoffatomen,Phenyl oder Tolyl bedeutet,

$R^3$ und $R^4$ gemeinsam unter Einbezug der Doppelbindung einen Phenyl oder Pyridylring ausbilden, der gegebenenfalls bis zu 2-fach gleich oder verschieden durch Hydroxy, Formyl, Carboxy, Fluor, Chlor, Brom, geradkettiges oder verzweigtes Acyl oder Alkoxycarbonyl mit jeweils bis zu 6 Kohlenstoffatomen, geradkettiges oder verzweigtes Perfluoralkyl mit bis zu 4 Kohlenstoffatomen oder durch geradkettiges oder verzweigtes Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann, oder durch die Gruppe der Formel $-CONR^8R^9$ substituiert sind,

worin

$R^8$ und $R^9$ die oben angegebene Bedeutung haben,

$R^5$ und $R^6$ gleich oder verschieden sind und für Wasserstoff, Fluor, Chlor, Brom, geradkettiges oder verzweigtes Alkyl mit bis zu 6 Kohlenstoffatomen, oder für geradkettiges oder verzweigtes Perfluoralkyl mit bis zu 4 Kohlenstoffatomen stehen,

$R^7$ für einen Rest der Formel

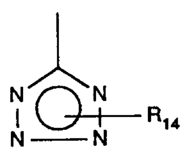

oder $CO-R^{15}$ steht,

worin

$R^{14}$ Wasserstoff, geradkettiges oder verzweigtes Alkyl mit bis zu 4 Kohlenstoffatomen oder Triphenylmethyl bedeutet

und

$R^{15}$ Hydroxy, geradkettiges oder verzweigtes Alkoxy mit bis zu 6 Kohlenstoffatomen, Phenoxy oder eine Gruppe der Formel $-NR^{16}R^{17}$ bedeutet,

worin

$R^{16}$ und $R^{17}$ gleich oder verschieden sind und Wasserstoff oder geradkettiges oder verzweigtes Alkyl mit bis zu 4 Kohlenstoffatomen bedeuten,

und deren Salze.

Besonders bevorzugt sind Verbindungen der allgemeinen Formel (I), in welcher

$R^1$ und $R^2$ gleich oder verschieden sind und für Wasserstoff, Cyano, Chlor oder für geradkettiges oder verzweigtes Alkyl mit bis zu 4 Kohlenstoffatomen stehen, das gegebenenfalls durch Cyclopropyl substituiert ist oder für Cyclopropyl stehen, oder für geradkettiges oder verzweigtes Acyl oder Alkoxycarbonyl mit jeweils bis zu 4 Kohlenstoffatomen, Benzyloxycarbonyl oder Carboxy stehen, oder für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder für eine Gruppe der Formel $-CO-NR^8R^9$, $BR^{10}$ oder $-NR^{11}R^{12}$ stehen,

worin

$R^8$ und $R^9$ gleich oder verschieden sind und Wasserstoff, Phenyl, Ethyl oder Benzyl bedeuten,

B ein Sauerstoff- oder Schwefelatom bedeutet,
$R^{10}$ geradkettiges oder verzweigtes Alkyl mit bis zu 4 Kohlenstoffatomen bedeutet,
$R^{11}$ und $R^{12}$ gleich oder verschieden sind und die oben angegebene Bedeutung von $R^8$ und $R^9$ haben oder $R^{11}$ oder $R^{12}$ die $-SO_2R^{13}$-Gruppe bedeutet,
worin
$R^{13}$ Methyl, Phenyl oder Tolyl bedeutet,
$R^3$ und $R^4$ gemeinsam unter Einbezug der Doppelbindung einen ankondensierten Phenyl- oder Pyridylring ausbilden, der gegebenenfalls bis zu 2-fach gleich oder verschieden durch Hydroxy, Carboxy, Fluor, Chlor, geradkettiges oder verzweigtes Acyl oder Alkoxycarbonyl mit jeweils bis zu 4 Kohlenstoffatomen oder geradkettiges oder verzweigtes Perfluoralkyl mit bis zu 3 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann
oder durch die Gruppe der Formel $-CO-NR^8R^9$ substituiert sind,
worin
$R^8$ und $R^9$ die oben angegebene Bedeutung haben,
$R^5$ und $R^6$ gleich oder verschieden sind und für Wasserstoff, Fluor, Chlor, geradkettiges oder verzweigtes Alkyl mit bis zu 4 Kohlenstoffatomen, oder für geradkettiges oder verzweigtes Perfluoralkyl mit bis zu 3 Kohlenstoffatomen stehen,
$R^7$ für einen Rest der Formel

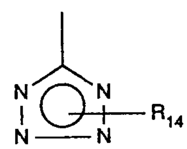

oder $CO-R^{15}$ steht,
worin
$R^{14}$ Wasserstoff, Methyl, Ethyl oder Triphenylmethyl bedeutet
und
$R^{15}$ Hydroxy, geradkettiges oder verzweigtes Alkoxy mit bis zu 4 Kohlenstoffatomen, Phenoxy oder eine Gruppe der Formel $-NR^{16}R^{17}$ bedeutet,
worin
$R^{16}$ und $R^{17}$ gleich oder verschieden sind und Wasserstoff oder geradkettiges oder verzweigtes Alkyl mit bis zu 3 Kohlenstoffatomen bedeuten

und deren Salze.

Außerdem wurde ein Verfahren zur Herstellung der erfindungsgemäßen Verbindungen der allgemeinen Formel (I) gefunden, dadurch gekennzeichnet, daß man

[A] Pyridone der allgemeinen Formel (II)

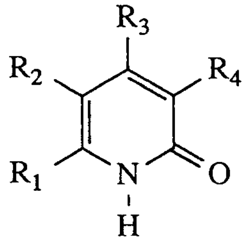

(II),

in welcher
$R^1$, $R^2$, $R^3$ und $R^4$ die oben angegebene Bedeutung haben,
mit Verbindungen der allgemeinen Formel (III)

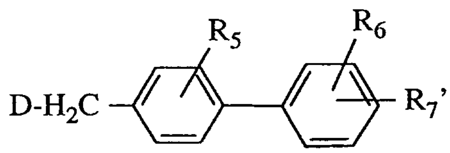 (III),

in welcher
$R^5$ und $R^6$ die oben angegebene Bedeutung haben,
D für Halogen, vorzugsweise für Brom steht
und
$R^{7'}$ für den Rest der Formel

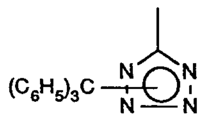

steht,
oder
[B] im Fall, daß $R^3$ und $R^4$ gemeinsam unter Einbeziehung der Doppelbindung einen gegebenenfalls substituierten Pyridylring bilden ($R^{3'}$,$R^{4'}$)
Verbindungen der allgemeinen Formel (IV)

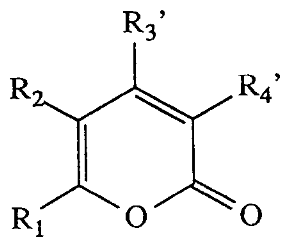 (IV),

in welcher
$R^1$, $R^3$, $R^3$ und $R^{4'}$ die oben angegebene Bedeutung haben,
mit Verbindungen der allgemeinen Formel (IIIa)

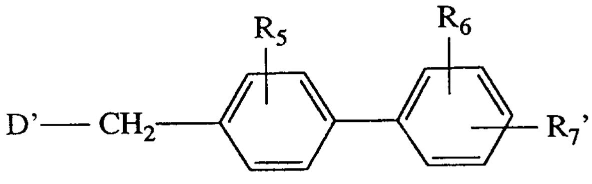 (IIIa),

in welcher
$R^5$ $R^6$ und $R^{7'}$ die oben angegebene Bedeutung haben,
und
D' für die $NH_2$-Gruppe steht,

in inerten Lösemitteln, gegebenenfalls in Anwesenheit einer Base, umsetzt und anschließend gegebenenfalls die Tritylgruppe mit Säuren abspaltet
und im Fall, daß $R^7 \neq H$, gegebenenfalls nach üblichen Methoden alkyliert oder verseift,
und gegebenenfalls die Substituenten $R^1$, $R^2$, $R^5$, $R^6$ und $R^7$ nach üblichen Methoden derivatisiert.

Das erfindungsgemäße Verfahren kann durch folgendes Formelschema beispielhaft erläutert werden:

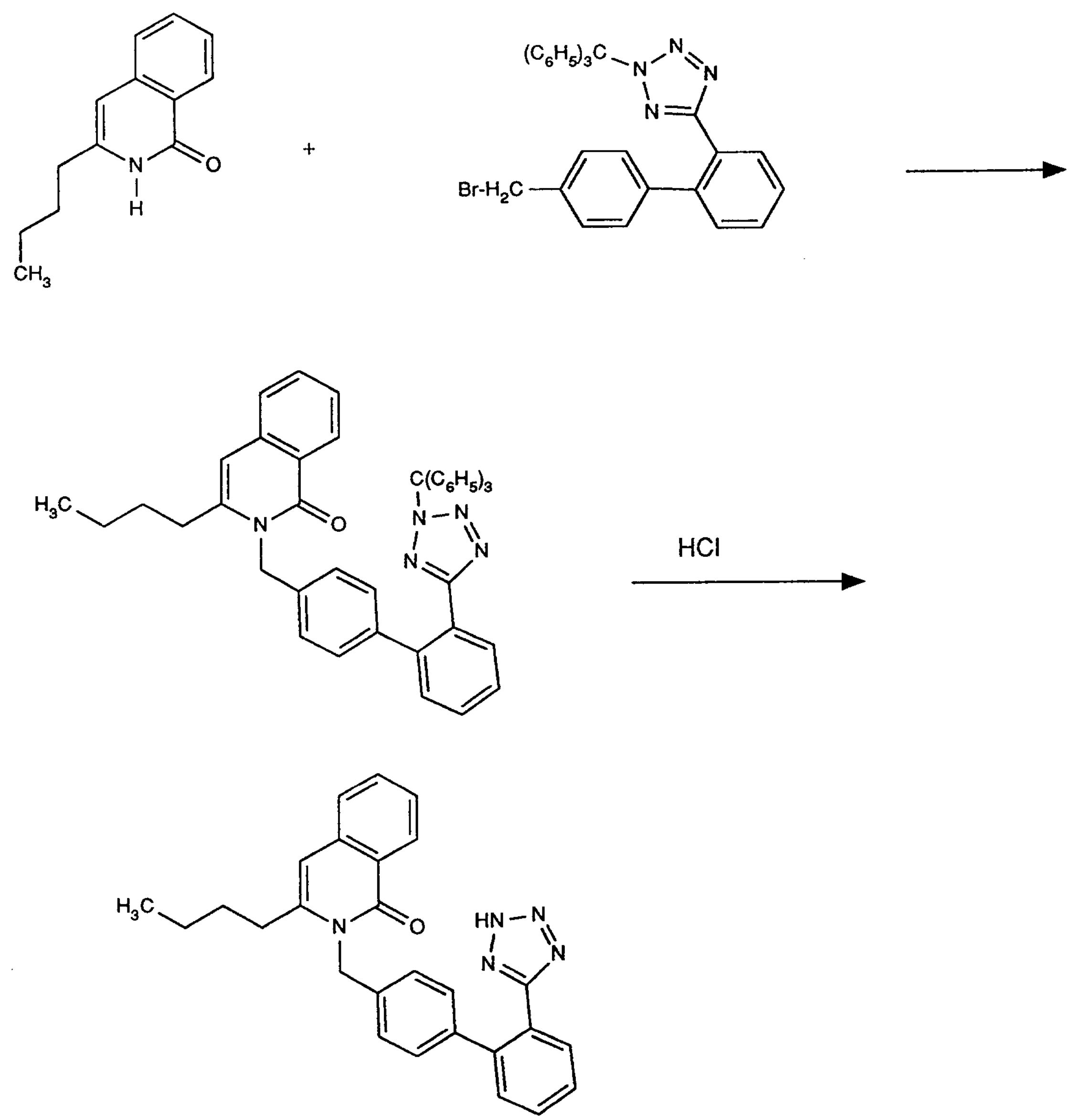


Als Lösemittel für die einzelnen Schritte der Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton, Nitromethan oder Dimethoxyethan. Ebenso ist es möglich, Gemische der genannten Lösemittel zu verwenden. Bevorzugt sind Tetrahydrofuran, Diethylether, Hexan, Essigester, Dioxan, Acetonitril und Dimethoxyethan.

Als Basen können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Caesiumcarbonat, Erdalkalicarbonate wie Calciumcarbonat, oder Alkali- oder Erdalkalialkoholate oder -amide wie Natrium- oder Kaliummethanolat, Natrium-oder Kaliumethanolat, Kalium-tert.butylat oder Kaliumamid, oder organische Amine (Trialkyl$(C_1-C_6)$amine) wie Triethylamin, oder Heterocyclen wie 1,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Triethylamin, Kaliumamid, Natriumhydroxid, Natriumcarbonat und Caesiumcarbonat.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30 °C bis +100 °C, bevorzugt von -10 °C bis +60 °C durchgeführt.

Dies erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Als Säuren für die Abspaltung der Triphenylmethylgruppe eignen sich im allgemeinen organische, gegebenenfalls halogenierte $C_1$-$C_6$-Carbonsäuren oder Protonensäuren. Bevorzugt sind Salzsäure, Eisessig oder Trifluoressigsäure.

Die Abspaltung erfolgt im allgemeinen in einem Temperaturbereich von 0 °C bis +120 °C, vorzugsweise von +20 °C bis +100 °C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man äquivalente Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1$-$C_6$)-Alkylhalogeniden, Sulfonsäureestern oder substituierten oder unsubstituierten ($C_1$-$C_6$)-Dialkyl- oder ($C_1$-$C_6$)Diarylsulfate, vorzugsweise Methyljodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Pyridone der allgemeinen Formel (II) sind neu und können im Fall, daß

[A] $R^3$ und $R^4$ einen Phenylring ausbilden, hergestellt werden, indem man beispielsweise Verbindungen der allgemeinen Formel (V)

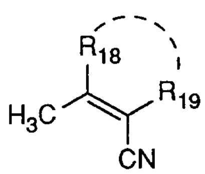

(V)

in welcher
$R^{18}$ und $R^{19}$ einen gegebenenfalls substituierten Phenylring bilden,
zunächst durch Umsetzungen mit Verbindungen der allgemeinen Formel (VI)

$R^{1'}$-$CO_2$-E (VI),

in welcher
$R^{1'}$ für einen Alkyl-, Alkenyl- oder Alkinyl-Rest steht,
und
E für $C_1$-$C_4$-Alkyl, vorzugsweise für Methyl steht,
unter Schutzgasatmosphäre, in einem der oben aufgeführten Lösemittel und in Anwesenheit einer der dort ebenfalls aufgeführten Basen,
vorzugsweise in Ammoniak mit Kaliumamid in die Verbindungen der allgemeinen Formel (VII)

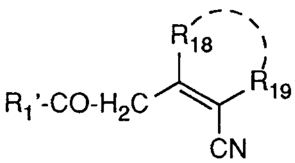

(VII),

in welcher
$R^{1'}$, $R^{18}$ und $R^{19}$ die oben angegebene Bedeutung haben,
überführt, anschließend mit Säuren in Alkoholen, vorzugsweise mit Schwefelsäure in Ethanol, cyclisiert, und im Fall, daß $R^1$ nicht für eine Alkyl-, Alkenyl- oder Alkinylgruppe steht, den Substituenten $R^{1'}$ nach üblichen Methoden derivatisiert;
[B] im Fall, daß $R^3$ und $R^4$ gemeinsam einen Pyridylring ausbilden, beispielsweise Pyridine der allgemeinen Formel (VIII)

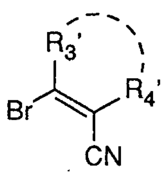

(VIII),

in welcher
$R^{3'}$ und $R^{4'}$ einen gegebenenfalls substituierten Pyridylring bilden,
zunächst durch Umsetzung mit Verbindungen der allgemeinen Formel (IX)

$HC \equiv C$-$R^{1'}$ (IX),

in welcher
$R^{1'}$ die oben angegebene Bedeutung hat,
im Autoklaven unter Schutzgasatmosphäre in Anwesenheit von Katalysatoren/Hilfsstoffen, vorzugsweise im System Bis-(triphenylphosphin)-Palladium(II)chlorid / Kupfer(I)iodid, in die Verbindungen der allgemeinen Formel (X)

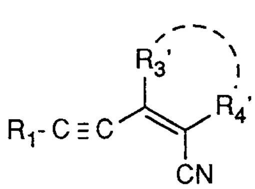

(X)

in welcher
$R^1$, $R^{3'}$ und $R^{4'}$ die oben angegebene Bedeutung haben,
überführt und anschließend entweder direkt wie unter [A] beschrieben cyclisiert oder zunächst über die Stufe der allgemeinen Formel (IVa)

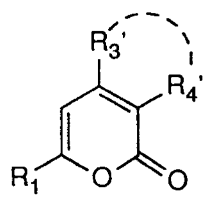

(IVa),

in welcher
$R^1$, $R^{3'}$ und $R^{4'}$ die oben angegebene Bedeutung haben,
cyclisiert und anschließend mit Ammoniak umsetzt,
und im Fall, daß $R^2 \neq H$ nach üblichen Methoden derivatisiert,
und ebenso die unter $R^3/R^4$ aufgeführten Phenyl- als auch die Pyridylsubstituenten gegebenfalls variiert.

Die Katalysatoren/Hilfsstoffe werden im allgemeinen in einer Menge von 0,001 mol bis 0,5 mol, bevorzugt von 0,01 mol bis 0,3 mol, jeweils bezogen auf 1 mol der Verbindungen der allgemeinen Formeln (V) und (VIII), eingesetzt.

Die Basen werden im allgemeinen in einer Menge von 1 mol bis 5 mol, bevorzugt von 1 mol bis 3 mol, jeweils bezogen auf 1 mol der Verbindungen der allgemeinen Formel (V), eingesetzt.

Die Reaktionstemperaturen für die einzelnen Schritte liegen in einem Bereich von 0°C bis 180°C, vorzugsweise von 20°C bis 150°C.

In Abhängigkeit der einzelnen Reaktionsschritte kann sowohl bei Normaldruck als auch bei erhöhtem Druck, beispielsweise 0,5 bis 5 bar, und gegebenenfalls unter Schutzgasatmosphäre gearbeitet werden.

Die Verbindungen der allgemeinen Formeln (V), (VI) und (VII) sind größtenteils bekannt oder können nach üblichen Methoden hergestellt werden (vgl. z.B. J.Org. Chem. 1966, 31, 3807).

Die Verbindungen der allgemeinen Formel (VIII) sind teilweise neu und können beispielsweise hergestellt werden, indem man zunächst die entsprechenden 3-Brom-substituierten Pyridine durch Umsetzung mit Wasserstoffperoxid in Essigsäure zu den jeweiligen Pyridin-N-oxiden umsetzt und in einem zweiten Schritt die Cyano-Gruppe nach üblichen Methoden, beispielsweise mit Trimethylsilylcyanid in Acetonitril und in Anwesenheit von Triethylamin, in einem Temperaturbereich von 20°C bis 120°C, vorzugsweise von 20°C bis 100°C einführt (vgl. hierzu J.Org. Chem. 1958, 23, 1616; Chem. Pharm. Bull. 1985, 33, 565).

Die Verbindungen der allgemeinen Formel (IX) sind an sich bekannt oder können nach üblichen Methoden hergestellt werden.

Die Verbindungen der allgemeinen Formeln (IV) und (IVa) sind teilweise bekannt oder neu und können dann beispielsweise wie oben beschrieben oder nach publizierten Methoden hergestellt werden (vgl. z.B. Chem. Pharm. Bull, 34(7), 2760-5; 33(2), 626-33; Heterocycles 32(5), 1310-16, Indian J.Chem., Sect. B, 20B(5), 376-9, Chem. Pharm. Bull. 1988, 36, 1890).

Die Verbindungen der allgemeinen Formel (III) sind größenteils bekannt. Die Verbindungen der allgemeinen Formel (IIIa) sind teilweise bekannt oder neu und können dann beispielsweise hergestellt werden, indem man Verbindungen der allgemeinen Formel (III) zunächst mit Lithiumazid in einem der oben aufgeführten Lösemitteln, vorzugsweise Dimethylformamid bei 70°C umsetzt und anschließend eine Reaktion mit Phosphortrichlorid/Tetrahydrofuran/Wasser bei Raumtemperatur durchführt.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an A II-Rezeptoren hemmen. Sie unterdrücken die vasokonstriktorischen und Aldosteronsekretionsstimulierenden Effekte des Angiotensin II. Darüberhinaus irhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37 °C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen (Applikationsvolumen pro Einzelgabe = 100 µl):

| | |
|---|---|
| KCl | 22,7;32,7;42,7;52,7 mmol/l |
| 1Noradrenalin | $3x10^{-9}$;$3x10^{-8}$;$3x10^{-7}$;$3x10^{-6}$ g/ml |
| Serotonin | $10^{-8}$;$10^{-7}$;$10^{-6}$;$10^{-5}$ g/ml |
| B-HT 920 | $10^{-7}$;$10^{-6}$;$10^{-5}$ g/ml |
| Methoxamin | $10^{-7}$;$10^{-6}$;$10^{-5}$ g/ml |
| Angiotensin II | $3x10^{-9}$;$10^{-8}$;$3x10^{-8}$;$10^{-7}$ g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

## Tabelle A:

**Hemmung der Gefäßkontraktion an isolierten Aortenringen von Kaninchen in vitro**

**$IC_{50}$ (g/ml) gegen Kontraktionen, induziert durch:**

| Bsp.Nr.: | AII |
|---|---|
| 2 | $2,3 \cdot 10^{-7}$ |
| 4 | $8,4 \cdot 10^{-7}$ |
| 6 | $>10^{-6}$ |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht. Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

| | |
|---|---|
| Bsp. 2 Ki = | 42 nM |
| Bsp. 4 Ki = | 750 nM |
| Bsp. 6 Ki = | 2000 nM |

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten oder Schweinen durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium mit Serumzuwachs, 2 mmol L-Glutamin und 15 mmol HEPES, pH 7,4 in 5% $CO_2$ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach

weiteren 4 Stunden der Einbau dieser Substanz in die TCA-präzipitierbare DNA der Zellen bestimmt.

| Bsp. | %-Hemmung bei $10^{-6}$ M |
|---|---|
| 6 | 70 % |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

2-(2-Oxo-hexyl)-benznitril

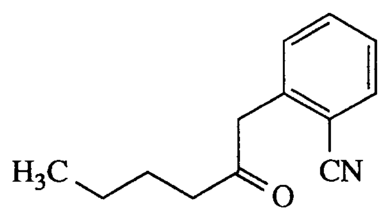

Unter Argon wird Kalium (3,8 g, 0,10 mol) in Ammoniak (150 ml) gelöst, mit einer Spatelspitze Eisen-III-nitrat versetzt und 15 min unter Rückfluß gerührt. Eine Lösung von 2-Toluolnitril (12 ml; 0,10 mol) in Ether (25 ml) wird zugetropft, des weiteren wird nach 10 min eine Losung von Valeriansäuremethylester (6,6 ml; 0,050 mol) in Ether (25 ml) zugefügt. Nach einer Stunde werden Ammoniumchlorid (6,1 g, 0,12 mol)) und Ether (25 ml) zugegeben, und über Nacht der Ammoniak abgedampft. Die Suspension wird kurz erhitzt, mit 6 N-Salzsäure sauer gestellt und mit Methylenchlorid extrahiert. Trocknen der organischen Phase über Natriumsulfat, Einengen und Kieselgelchromatographie (Hexan : Essigester = 5:1) liefern 3,1 g eines gelben Öls (31% der Theorie).
$R_f$ = 0,52 (Hexan : Ethylacetat = 3 : 1).

Beispiel II

3-Butyl-isochinolin-1(2H)-on

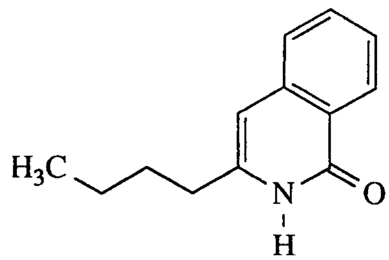

Unter Eiskühlung wird zu einer Lösung von Beispiel I (3,1 g; 15 mmol) in Ethanol/Wasser (19:1; 600 ml) konzentrierte Schwefelsäure (60 ml) gegeben. Nach 7 h Erhitzen zum Rückfluß wird die Reaktionslösung auf Eis gegossen und eingeengt. Absaugen und Umkristallisation des ausgefallenen Produkts aus Hexan ergeben 1,8 g eines weißen Feststoffs (57% der Theorie).
Fp.: 137 °C
$R_f$ = 0,28 (Hexan : Essigester = 3:1)

Beispiel III

2-(2-Oxo-butyl)-benznitril

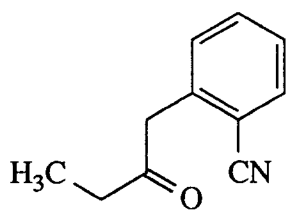

Analog zur Beispiel I werden durch Acylierung von 2-Tolylnitril (1 ml; 0,10 mol) mit Propionsäuremethylester (4,8 ml; 50 mmol) 3,1 g eines gelben Öls erhalten (36% der Theorie).
$R_f$ = 0,46 (Hexan : Essigester = 3:1)

Beispiel IV

3-Ethyl-isochinolin-1(2H)-on

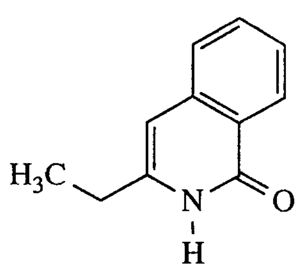

Analog Beispiel II werden aus Beispiel III (3,1 g; 18 mmol) 1,6 g eines Feststoffs erhalten (52% der Theorie).

Fp.: 136 °C
$R_f$ = 0,13 (Hexan : Essigester = 3:1)

Beispiel V

2-(Benzoylmethyl)-benznitril

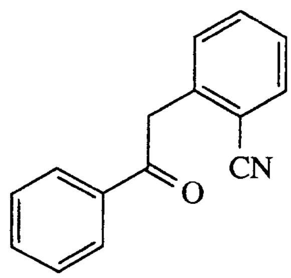

Analog zur Beispiel I werden durch Acylierung von 2-Tolylnitril (12 ml; 0,10 mol) mit Benzoesäuremethylester (6,3 ml; 50 mmol) 3,1 g eines weißen Feststoffs erhalten (50% der Theorie).
Fp.: 109 °C
$R_f$ = 0,42 (Hexan : Essigester = 3:1)

Beispiel VI

3-Phenyl-isochinolin-1(2H)-on

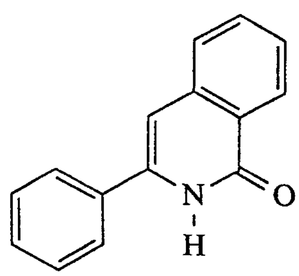

Analog Beispiel II werden aus Beispiel V (4,3 g; 19 mmol) 2,0 g eines Feststoffs erhalten (46% der Theorie).
Fp.: 105 °C
$R_f$ = 0,15 (Hexan : Essigester = 3:1)

Beispiel VII

3-Brompyridin-N-oxid

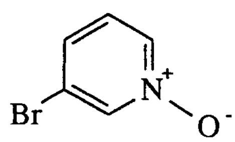

Eine Losung von 3-Brompyridin (3 ml; 0,32 mol) in Eisessig (250 ml) wird mit Wasserstoffperoxid: $H_2O$ (30:70; 50 ml) versetzt und bei 100 °C gerührt. Nach 3 h und 19 h wird weiteres Wasserstoffperoxid:$H_2O$

(30:70; je 25 ml) zugegeben und noch 4 h auf 100°C erhitzt. Die Reaktionslösung wird auf ein Drittel des Volumens eingeengt, mit Wasser wieder aufgefüllt und vollständig eingeengt. Der Rückstand wird in Methylenchlorid gelöst und mit Natriumcarbonatlösung gewaschen. Sättigen der waßrigen Phase mit Kochsalz, Extraktion mit Methylenchlorid sowie Trocknen und Einengen der vereinigten organischen Phasen liefern 43 g eines Öls (77% der Theorie).
$R_f$ = 0,37 (Methylenchlorid : Methanol = 20:1)

Beispiel VIII

3-Brom-2-cyanpyridin

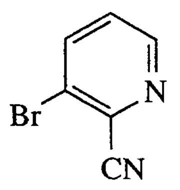

Eine Lösung von Beispiel VII (22 g; 0,12 mol), Trimethylsilylcyanid (45 ml; 0,36 mmol) und Triethylamin (33 ml; 0,24 mol) in Acetonitril (120 ml) wird 4 h zum Rückfluß erhitzt, eingeengt und auf 3N-Natriumcarbonat-Lösung gegossen. Extraktion mit Methylenchlorid sowie Trocknen und Einengen der organischen Phasen liefert nach Umkristallisation aus Hexan / Ethylacetat 17 g eines Feststoffs (79% der Theorie).
Fp.: 92°C
$R_f$ = 0,31 (Hexan : Essigester = 3:1)

Beispiel IX

2-Cyan-3-hex-1-inylpyridin

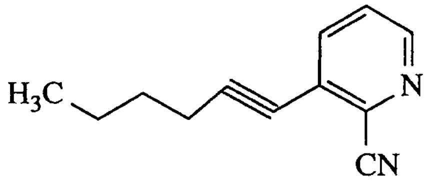

Im Autoklaven werden die Verbindung aus Beispiel VIII (4,8 g; 26 mmol), 1-Hexin (3,6 ml; 31 mmol), Bis-(triphenylphosphin)-Palladium(II)-chlorid (0,42 g; 0,60 mmol) und Kupfer(I)-jodid (0,21 g; 1,1 mmol) mit Stickstoff gespült und 5 h auf 120°C erhitzt. Verteilung des Reaktionsgemisches zwischen Wasser und Ether, sowie Trocknen und Einengen der organischen Phase liefert nach Kieselgelchromatographie (Hexan : Essigester = 4:1) 0,76 g eines Öls (16% der Theorie).
$R_f$ = 0,60 (Hexan : Essigester = 3:1)

Beispiel X

6-Butyl-pyrano[3,4-b]pyridin-8-on

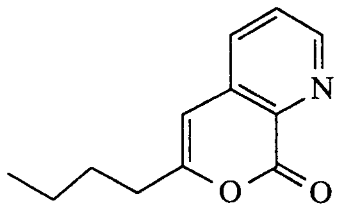

Die Verbindung aus Beispiel IX (100 mg; 0,490 mmol) wird in Propanphosphorsäureanhydrid (2 g) 3 h auf 130 °C erhitzt. Das Reaktionsgemisch wird auf Eiswasser gegeben und mit Kaliumcarbonat basisch gestellt. Extraktion der wäßrigen Phase mit Chloroform, Trocknen der organischen Phase über Natriumsulfat, Einengen und Kieselgelchromatographie (Dichlormethan: Methanol = 50:1-40:1) ergeben 76 mg eines Harzes (76 % der Theorie).
$R_f$ = 0,58 (Dichlormethan:Methanol = 20:1).

Beispiel XI

[2'-(N-Triphenylmethyl-tetrazol-5-yl)-biphenyl-4-yl]methylazid

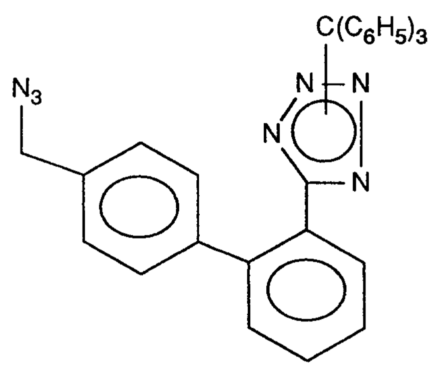

Eine Losung von [2'-(N-Triphenylmethyl-tetrazol-5-yl)-biphenyl-4-yl]methylbromid (5 g, 9 mmol) in Dimethylformamid (40 ml) wird mit Lithiumazid (0,88 g; 18 mmol) versetzt und 15 min im vorgeheizten Ölbad bei 70 °C gerührt. Das Lösungsmittel wird im Vakuum abgezogen, der Rückstand zwischen Wasser und Essigester verteilt, die organische Phase mit Wasser und gesättigtem Kochsalz gewaschen und über Natriumsulfat getrocknet. Einengen ergibt 4,5 g fahlgelben Feststoffs (96 % der Theorie).
$R_f$ = 0,86 (Hexan : Essigester = 5:1, DC zweimal laufen lassen).

Beispiel XII

[2'-(N-Triphenylmethyl-tetrazol-5-yl)-biphenyl-4-yl]methylamin

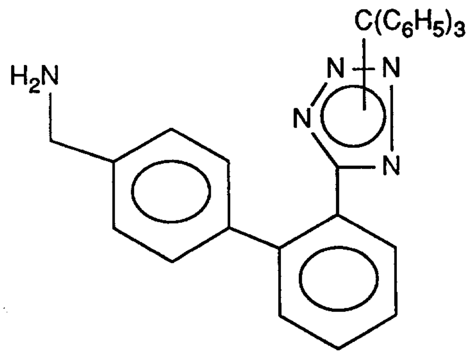

Eine Lösung der Verbindung aus Beispiel XI (2,0 g; 3,8 mmol) in Tetrahydrofuran (100 ml) wird mit Triphenylphosphin (1,2 g; 4,6 mmol) und Wasser (0,10 ml; 5,8 mmol) versetzt und 4 h bei Raumtemperatur gerührt. Einengen und Kieselgelchromatographie (Dichlormethan:Methanol = 20:1) des Rückstandes ergeben 0,78 g eines fahlgelben Harzes (41 % der Theorie).
$R_f$ = 0,16 (Dichlormethan:Methanol = 20:1).

Herstellungsbeispiele

Beispiel 1

3-Butyl-2-{[2'-(N-triphenylmethyl-tetrazol-5-yl)-biphenyl-4-yl]methyl}-isochinolin-1(2H)-on

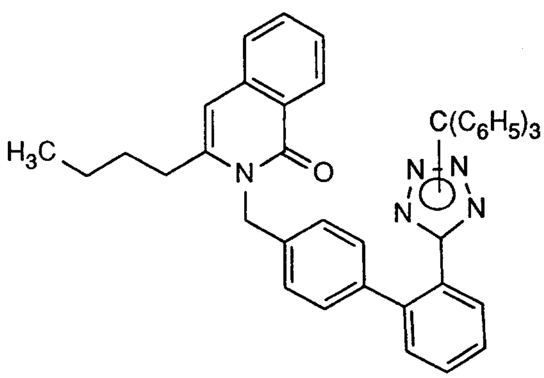

Eine Lösung von Beispiel II (1,0 g; 5,0 mmol) in Dimethylsulfoxid (20 ml) wird mit [2'-(N-Triphenylmethyl-tetrazol-5-yl)-biphenyl-4-yl]methylbromid (3,1 g; 5,5 mmol) und Caesiumcarbonat (1,4 g; 2,5 mmol) versetzt und 3 h bei 80 °C gerührt. Nach Zugabe von weiterem Bromid (1,4 g; 2,5 mmol) und Caesiumcarbonat (0,82 g; 2,5 mmol) und 4 h Rühren bei 80 °C wird die Reaktionslösung zwischen Essigester und Wasser verteilt. Die organische Phase wird mit Wasser gewaschen, getrocknet und eingeengt. Kieselgelchromatographie (Hexan : Essigester = 4:1) ergibt 0,89 g Produkt (26% der Theorie).
$R_f$ = 0,31 (Hexan : Essigester = 3:1)

Beispiel 2

3-Butyl-2-{[2'-(tetrazol-5-yl)-biphenyl-4-yl]methyl}-isochinolin-1(2H)-on

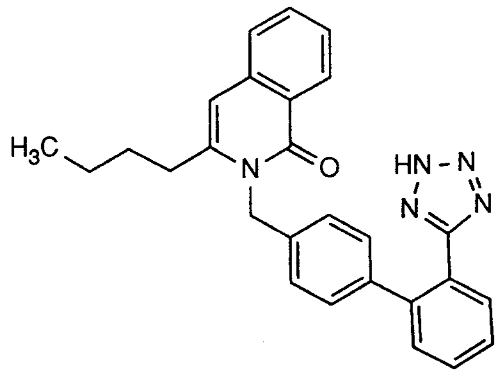

(0,18 g; 0,27 mmol) der Verbindung aus Beispiel 1 werden mit 4N-HCl / Dioxan gelöst und 2 h gerührt. Nach dem Alkalisch-stellen mit konzentrierter Natronlauge wird die Reaktionslösung eingeengt und mit Ether extrahiert. Die wäßrige Phase wird auf pH 2 angesäuert, mit Kochsalz gesättigt und mit Essigester extrahiert. Trocknen und Einengen der organischen Phasen liefern 70 mg eines Harzes (59% der Theorie).
$R_f$ = (Methylenchlorid : Methanol 20:1)

Beispiel 3

3-Ethyl-2-{[2'-(N-triphenylmethyl-tetrazol-5-yl)-biphenyl-4-yl]-methyl]-isochinolin-1(2H)-on

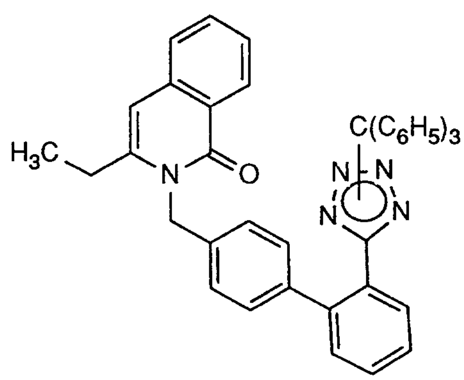

Analog Beispiel 1 werden aus Beispiel IV (1,0 g; 5,8 mmol); 1,18 g eines Feststoffes erhalten (31% der Theorie).
$R_f$ = 0,27 (Hexan : Ethylacetat = 3:1)

Beispiel 4

3-Ethyl-2-{[2'-(tetrazol-5-yl)-biphenyl-4-yl]methyl}-isochinolin-1(2H)-on

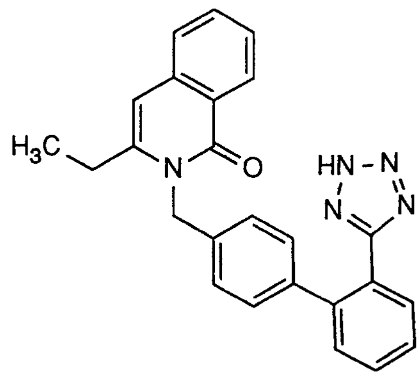

Analog Beispiel 2 werden aus Beispiel 3 (0,26 g; 0,38 mmol) 95 mg eines Harzes erhalten (61% der Theorie).
$R_f$ = (Methylenchlorid : Methanol = 20:1)

Beispiel 5

3-Phenyl-2-{[2'-(2-triphenylmethyl-tetrazol-5-yl)-biphenyl-4-yl]methyl}-isochinolin-1(2H)-on

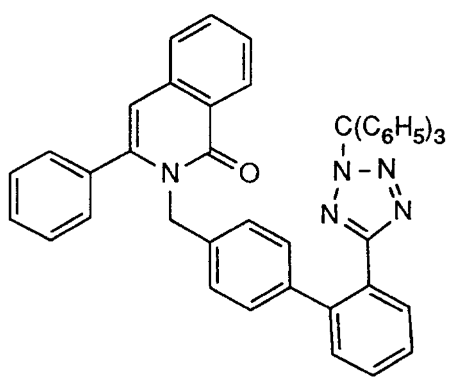

Analog Beispiel 1 werden aus Beispiel VI (1,0 g; 4,5 mmol) 0,56 g eines Feststoffs erhalten (14% der Theorie).
$R_f$ = 0,25 (Hexan : Essigester = 3:1)

Beispiel 6

3-Phenyl-2-{[2'-(tetrazol-5-yl)-biphenyl-4-yl]methyl]-isochinolin-1(2H)-on

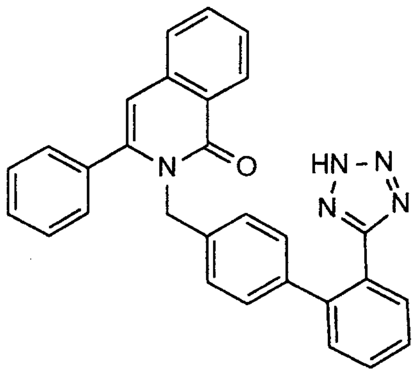

Analog Beispiel 2 werden aus Beispiel 5 (0,53 g; 0,76 mmol) 0,16 g eines Harzes erhalten (45% der Theorie).
$R_f$ = 0,35 (Methylenchlorid : Methanol = 20:1)

Beispiel 7

3-Chlor-5,8-dimethyl-2-{[2'-(N-triphenylmethyl-tetrazol-5-yl)biphenyl-4-yl]methyl}-isochinolin-1(2H)-on

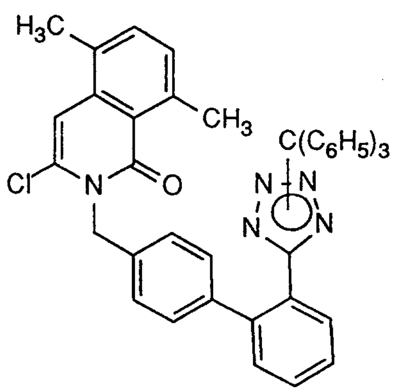

Analog Beispiel 1 werden aus 3-Chlor-5,8-dimethyl-isochinolin-1(2H)-on (1,9 g; 9,0 mmol) 0,32 g eines Feststoffs erhalten (24% der Theorie).
$R_f$ = 0,60 (Hexan : Essigester = 3:1)

Beispiel 8

3-Chlor-5,8-dimethyl-2-{[2'-(tetrazol-5-yl)-biphenyl-4-yl]methyl}-isochinolin-1(2H)-on

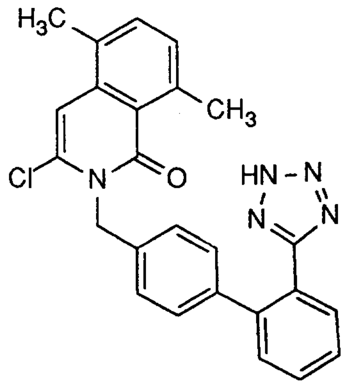

Analog Beispiel 2 werden aus Beispiel 9 (0,20 g; 0,29 mmol) 76 mg eines Harzes erhalten (59% der Theorie).
$R_f$ = 0,57 (Methylenchlorid : Methanol = 20:1)

Beispiel 9

6-Butyl-7-{[2'-(N-triphenylmethyl-tetrazol-5-yl)-biphenyl-4-yl]methyl}-1,7-naphthyridin-8[7H)-on

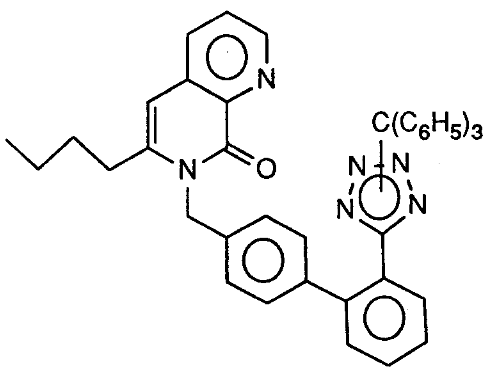

Eine Lösung der Verbindung aus Beispiel X (161 mg; 0,792 mmol) in THF (5 ml) wird mit der Verbindung aus Beispiel XII (469 mg; 0,950 mmol) versetzt und 2 h zum Rückfluß erhitzt. Einengen des Reaktionsgemisches und Verteilen des Rückstandes zwischen Essigester und IM-Kaliumhydrogensulfat-Lösung, Trocknen der organischen Phase über Natriumsulfat, Einengen und Kieselgelchromatographie (Hexan:Essigester = 3:1) ergeben 101 mg eines weißen Feststoffs (19 % der Theorie).
$R_f$ = 0,40 (Hexan:Essigester = 2:1).

Beispiel 10

6-Butyl-7-{[2'-(tetrazol-5-yl)-biphenyl-4-yl]methyl}-1,7-naphthyridin-8(7H)-on

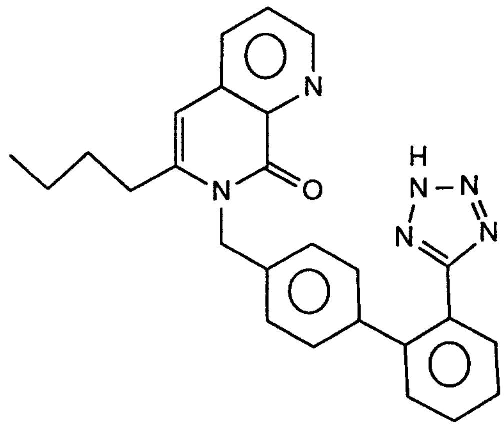

Analog Beispiel 2 werden aus der Verbindung aus Beispiel 9 (70 mg; 0,10 mmol) 34 mg eines Feststoffs erhalten (77 % der Theorie).
$R_f$ = 0,20 (Dichlormethan:Methanol:Essigsäure = 100:10:1).

## Patentansprüche

**1.** Biphenylmethyl-substituierte Pyridone der allgemeinen Formel

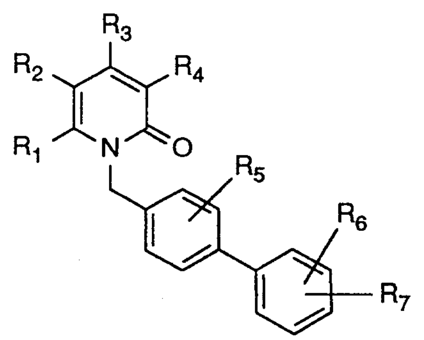

(I),

in welcher

| | |
|---|---|
| $R^1$ und $R^2$ | gleich oder verschieden sind und für Wasserstoff, Cyano, Halogen oder<br>für geradkettiges oder verzweigtes Alkyl, Alkenyl oder Alkinyl mit jeweils bis zu 8 Kohlenstoffatomen stehen, die gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen oder Phenyl substituiert sind, oder<br>für Cycloalkyl mit 3 bis 6 Kohlenstoffatomen stehen,<br>für geradkettiges oder verzweigtes Acyl oder Alkoxycarbonyl mit jeweils bis zu 8 Kohlenstoffatomen, Benzyloxycarbonyl oder Carboxy stehen, oder<br>für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder<br>für die Gruppe der Formel $-CO-NR^8R^9$, $B-R^{10}$ oder $-NR^{11}R^{12}$ stehen,<br>worin |
| $R^8$ und $R^9$ | gleich oder verschieden sind und Wasserstoff, Phenyl, geradkettiges oder verzweig- |

tes Alkyl mit bis zu 6 Kohlenstoffatomen oder Benzyl bedeuten,

B ein Sauerstoff- oder Schwefelatom bedeutet,

$R^{10}$ geradkettiges oder verzweigtes Alkyl mit bis zu 8 Kohlenstoffatomen bedeutet,

$R^{11}$ und $R^{12}$ gleich oder verschieden sind und die oben angegebene Bedeutung von $R^8$ und $R^9$ haben oder $R^{11}$ oder $R^{12}$ die $-SO_2R^{13}$-Gruppe bedeutet,

worin

$R^{13}$ geradkettiges oder verzweigtes Alkyl mit bis zu 6 Kohlenstoffatomen, Benzyl oder Phenyl bedeutet, die gegebenenfalls durch Methyl substituiert sind,

$R^3$ und $R^4$ unter Einbezug der Doppelbindung einen Phenyl- oder Pyridylring ausbilden, der gegebenenfalls bis zu 3-fach gleich oder verschieden durch Hydroxy, Formyl, Carboxy, Halogen, geradkettiges oder verzweigtes Acyl oder Alkoxycarbonyl mit jeweils bis zu 8 Kohlenstoffatomen, geradkettiges oder verzweigtes Perfluoralkyl mit bis zu 6 Kohlenstoffatomen oder durch geradkettiges oder verzweigtes Alkyl mit bis zu 8 Kohlenstoffatomen substituiert ist, das seinerseits durch Hydroxy oder geradkettiges oder verzweigtes Alkoxy mit bis zu 6 Kohlenstoffatomen substituiert sein kann, oder durch die Gruppe der Formel $-CONR^8R^9$ substituiert sind,

worin

$R^8$ und $R^9$ die oben angegebene Bedeutung haben,

$R^5$ und $R^6$ gleich oder verschieden sind und für Wasserstoff, Halogen oder geradkettiges oder verzweigtes Alkyl mit bis zu 8 Kohlenstoffatomen, oder

für geradkettiges oder verzweigtes Perfluoralkyl mit bis zu 6 Kohlenstoffatomen stehen,

$R^7$ für einen Rest der Formel

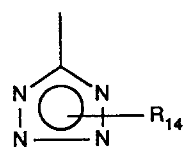

oder oder $CO-R^{15}$ steht,

worin

$R^{14}$ Wasserstoff, geradkettiges oder verzweigtes Alkyl mit bis zu 6 Kohlenstoffatomen oder Triphenylmethyl bedeutet

und

$R^{15}$ Hydroxy, geradkettiges oder verzweigtes Alkoxy mit bis zu 8 Kohlenstoffatomen, Phenoxy oder eine Gruppe der Formel $-NR^{16}R^{17}$ bedeutet,

worin

$R^{16}$ und $R^{17}$ gleich oder verschieden sind und Wasserstoff oder geradkettiges oder verzweigtes Alkyl mit bis zu 6 Kohlenstoffatomen oder Phenyl bedeuten,

und deren Salze.

**2.** Biphenylmethyl-substituierte Pyridone nach Anspruch 1, wobei

$R^1$ und $R^2$ gleich oder verschieden sind und für Wasserstoff, Cyano, Chlor oder

für geradkettiges oder verzweigtes Alkyl, Alkenyl oder Alkinyl mit jeweils bis zu 6 Kohlenstoffatomen stehen, die gegebenenfalls durch Cyclopropyl, Cyclopentyl, Cyclohexyl, Hydroxy oder durch geradkettiges oder verzweigtes Alkoxy mit bis zu 4 Kohlenstoffatomen oder Phenyl substituiert sind, oder

für Cyclopropyl, Cyclopentyl oder Cyclohexyl stehen,

für geradkettiges oder verzweigtes Acyl oder Alkoxycarbonyl mit jeweils bis zu 6 Kohlenstoffatomen, Benzyloxycarbonyl oder Carboxy stehen, oder

für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder

für eine Gruppe der Formel $-CO-NR^8R^9$, $B-R^{10}$ oder $-NR^{11}R^{12}$ stehen,

worin

| | |
|---|---|
| $R^8$ und $R^9$ | gleich oder verschieden sind und Wasserstoff, Phenyl, geradkettiges oder verzweigtes Alkyl mit bis zu 4 Kohlenstoffatomen oder Benzyl bedeuten, |
| B | ein Sauerstoff- oder Schwefelatom bedeutet, |
| $R^{10}$ | geradkettiges oder verzweigtes Alkyl mit bis zu 6 Kohlenstoffatomen bedeutet, |
| $R^{11}$ und $R^{12}$ | gleich oder verschieden sind und die oben angegebene Bedeutung von $R^8$ und $R^9$ haben oder $R^{11}$ oder $R^{12}$ die $-SO_2R^{13}$-Gruppe bedeutet, worin |
| $R^{13}$ | geradkettiges oder verzweigtes Alkyl mit bis zu 4 Kohlenstoffatomen,Phenyl oder Tolyl bedeutet, |
| $R^3$ und $R^4$ | gemeinsam unter Einbezug der Doppelbindung einen Phenyl oder Pyridylring ausbilden, der gegebenenfalls bis zu 2-fach gleich oder verschieden durch Hydroxy, Formyl, Carboxy, Fluor, Chlor, Brom, geradkettiges oder verzweigtes Acyl oder Alkoxycarbonyl mit jeweils bis zu 6 Kohlenstoffatomen, geradkettiges oder verzweigtes Perfluoralkyl mit bis zu 4 Kohlenstoffatomen oder durch geradkettiges oder verzweigtes Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann, oder durch die Gruppe der Formel $-CONR^8R^9$ substituiert sind, worin |
| $R^8$ und $R^9$ | die oben angegebene Bedeutung haben, |
| $R^5$ und $R^6$ | gleich oder verschieden sind und für Wasserstoff, Fluor, Chlor, Brom, geradkettiges oder verzweigtes Alkyl mit bis zu 6 Kohlenstoffatomen, oder für geradkettiges oder verzweigtes Perfluoralkyl mit bis zu 4 Kohlenstoffatomen stehen, |
| $R^7$ | für einen Rest der Formel 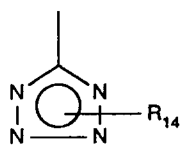 oder $CO-R^{15}$ steht, worin |
| $R^{14}$ | Wasserstoff, geradkettiges oder verzweigtes Alkyl mit bis zu 4 Kohlenstoffatomen oder Triphenylmethyl bedeutet und |
| $R^{15}$ | Hydroxy, geradkettiges oder verzweigtes Alkoxy mit bis zu 6 Kohlenstoffatomen, Phenoxy oder eine Gruppe der Formel $-NR^{16}R^{17}$ bedeutet, worin |
| $R^{16}$ und $R^{17}$ | gleich oder verschieden sind und Wasserstoff oder geradkettiges oder verzweigtes Alkyl mit bis zu 4 Kohlenstoffatomen bedeuten, |

und deren Salze.

**3.** Biphenylmethyl-substituierte Pyridone nach Anspruch 1, wobei

| | |
|---|---|
| $R^1$ und $R^2$ | gleich oder verschieden sind und für Wasserstoff, Cyano, Chlor oder für geradkettiges oder verzweigtes Alkyl mit bis zu 4 Kohlenstoffatomen stehen, das gegebenenfalls durch Cyclopropyl substituiert ist oder für Cyclopropyl stehen, oder für geradkettiges oder verzweigtes Acyl oder Alkoxycarbonyl mit jeweils bis zu 4 Kohlenstoffatomen, Benzyloxycarbonyl oder Carboxy stehen, oder für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder für eine Gruppe der Formel $-CO-NR^8R^9$, $B-R^{10}$ oder $-NR^{11}R^{12}$ stehen, worin |

| | |
|---|---|
| $R^8$ und $R^9$ | gleich oder verschieden sind und Wasserstoff, Phenyl, Ethyl oder Benzyl bedeuten, |
| B | ein Sauerstoff- oder Schwefelatom bedeutet, |
| $R^{10}$ | geradkettiges oder verzweigtes Alkyl mit bis zu 4 Kohlenstoffatomen bedeutet, |
| $R^{11}$ und $R^{12}$ | gleich oder verschieden sind und die oben angegebene Bedeutung von $R^8$ und $R^9$ haben oder $R^{11}$ oder $R^{12}$ die $-SO_2R^{13}$-Gruppe bedeutet, worin |
| $R^{13}$ | Methyl, Phenyl oder Tolyl bedeutet, |
| $R^3$ und $R^4$ | gemeinsam unter Einbezug der Doppelbindung einen ankondensierten Phenyl- oder Pyridylring ausbilden, der gegebenenfalls bis zu 2-fach gleich oder verschieden durch Hydroxy, Carboxy, Fluor, Chlor, geradkettiges oder verzweigtes Acyl oder Alkoxycarbonyl mit jeweils bis zu 4 Kohlenstoffatomen oder geradkettiges oder verzweigtes Perfluoralkyl mit bis zu 3 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann, oder durch die Gruppe der Formel $-CO-NR^8R^9$ substituiert sind, worin |
| $R^8$ und $R^9$ | die oben angegebene Bedeutung haben, |
| $R^5$ und $R^6$ | gleich oder verschieden sind und für Wasserstoff, Fluor, Chlor, geradkettiges oder verzweigtes Alkyl mit bis zu 4 Kohlenstoffatomen, oder für geradkettiges oder verzweigtes Perfluoralkyl mit bis zu 3 Kohlenstoffatomen stehen, |
| $R^7$ | für einen Rest der Formel |

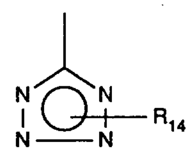

oder oder $CO-R^{15}$ steht,
worin

| | |
|---|---|
| $R^{14}$ | Wasserstoff, Methyl, Ethyl oder Triphenylmethyl bedeutet und |
| $R^{15}$ | Hydroxy, geradkettiges oder verzweigtes Alkoxy mit bis zu 4 Kohlenstoffatomen, Phenoxy oder eine Gruppe der Formel $-NR^{16}R^{17}$ bedeutet, worin |
| $R^{16}$ und $R^{17}$ | gleich oder verschieden sind und Wasserstoff oder geradkettiges oder verzweigtes Alkyl mit bis zu 3 Kohlenstoffatomen bedeuten |

und deren Salze.

**4.** Biphenylmethyl-substituierte Pyridone nach Anspruch 1 zur therapeutischen Anwendung.

**5.** Verfahren zur Herstellung von Biphenylmethyl-substituierten Pyridonen nach Anspruch 1, dadurch gekennzeichnet, daß man

[A] Pyridone der allgemeinen Formel (II)

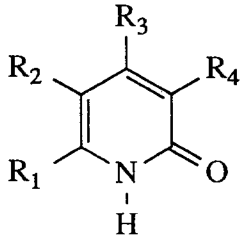

(II)

in welcher

$R^1$, $R^2$, $R^3$ und $R^4$ die oben angegebene Bedeutung haben,
mit Verbindungen der allgemeinen Formel (III)

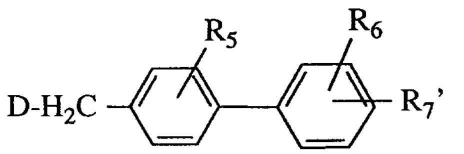

(III),

in welcher
$R^5$ und $R^6$ die oben angegebene Bedeutung haben,
D für Halogen, vorzugsweise für Brom steht
und
$R^{7'}$ für den Rest der Formel

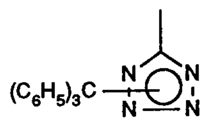

steht,
[B] im Fall, daß $R^3$ und $R^4$ gemeinsam unter Einbeziehung der Doppelbindung einen gegebenenfalls substituierten Pyridylring bilden ($R^{3'}$,$R^{4'}$)
Verbindungen der allgemeinen Formel (IV)

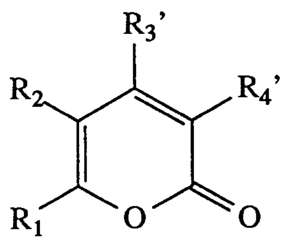

(IV),

in welcher
$R^1$, $R^3$, $R^{3'}$ und $R^{4'}$ die oben angegebene Bedeutung haben,
mit Verbindungen der allgemeinen Formel (IIIa)

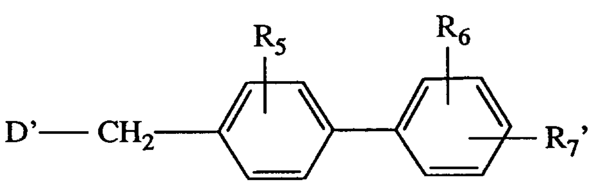

(IIIa),

in welcher
$R^5$, $R^6$ und $R^{7'}$ die oben angegebene Bedeutung haben,
und
D' für die $NH_2$-Gruppe steht,
in inerten Losemitteln, gegebenenfalls in Anwesenheit einer Base, umsetzt und anschließend gegebenenfalls die Tritylgruppe mit Säuren abspaltet
und im Fall, daß $R^7 \neq H$, gegebenenfalls nach üblichen Methoden alkyliert oder verseift,
und gegebenenfalls die Substituenten $R^1$, $R^2$, $R^5$, $R^6$ und $R^7$ nach üblichen Methoden derivatisiert.

6. Verfahren nach Anspruch 5, dadurch gekennzeichnet, daß die Umsetzung in einem Temperaturbereich von -30 °C bis +100 °C durchgeführt wird.

7. Arzneimittel enthaltend mindestens ein Biphenylmethyl-substituiertes Pyridon nach Anspruch 1.

8. Arzneimittel nach Anspruch 7 zur Behandlung von arterieller Hypertonie und Atherosklerose.

9. Verwendung von Biphenylmethyl-substituierten Pyridonen nach Anspruch 1 zur Herstellung von Arzneimitteln.

10. Verwendung nach Anspruch 9 zur Herstellung von Arzneimitteln zur Behandlung von arterieller Hypertonie und Atherosklerose.

Europäisches Patentamt

# EUROPÄISCHER RECHERCHENBERICHT

Nummer der Anmeldung

EP 93 10 6987

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,Y | EP-A-0 487 745 (MEIJI SEIKA KABUSHIKI KAISHA)<br>3. Juni 1992<br>* Anspruch 1 *<br>--- | 1-10 | C07D401/10<br>A61K31/47 |
| P,Y | EP-A-0 500 297 (FISONS PLC)<br>26. August 1992<br>* Anspruch 1 *<br>--- | 1-10 | |
| P,Y | EP-A-0 530 702 (MERCK PATENT GESSELLSCHAFT)<br>10. März 1993<br>* Anspruch 1 *<br>--- | 1-10 | |
| A | EP-A-0 154 190 (MERCK PATENT GESELLSCHAFT)<br>11. September 1985<br>* Anspruch 1 *<br>----- | 1-10 | |

RECHERCHIERTE SACHGEBIETE (Int. Cl.5 )

C07D
A61K

Der vorliegende Recherchenbericht wurde für alle Patentansprüche erstellt

| Recherchenort | Abschlußdatum der Recherche | Prüfer |
|---|---|---|
| MUENCHEN | 30 JUNI 1993 | GETTINS M.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)